(19) Europäisches Patentamt
European Patent Office
Office européen des brevets

(11) Publication number: 0 339 496 A2

# (12) EUROPEAN PATENT APPLICATION

(21) Application number: 89107168.0

(22) Date of filing: 20.04.89

(51) Int. Cl.4: C07C 133/10 , C07D 295/12 , C07D 211/46 , C07D 213/81 , C07D 217/04 , A61K 31/155 , A61K 31/395 , A61K 31/34

Claims for the following Contracting States: ES + GR

(30) Priority: 26.04.88 JP 101365/88

(43) Date of publication of application: 02.11.89 Bulletin 89/44

(84) Designated Contracting States: AT BE CH DE ES FR GB GR IT LI LU NL SE

(71) Applicant: ONO PHARMACEUTICAL CO., LTD.
1-5, Doshomachi 2-chome Chuo-ku
Osaka-shi Osaka(JP)

(72) Inventor: Ohuchida, Shuichi Ono Pharmaceutical Co., Ltd.
Minase Res. Inst. 3-1-1 Sakurai
Shimamoto-cho
Mishima-gun Osaka(JP)
Inventor: Toda, Masaaki Ono Pharmaceutical Co., Ltd.
Minase Res. Inst. 3-1-1 Sakurai
Shimamoto-cho
Mishima-gun Osaka(JP)
Inventor: Miyamoto, Tsumoru Ono Pharmaceutical Co., Ltd.
Minase Res. Inst. 3-1-1 Sakurai
Shimamoto-cho
Mishima-gun Osaka(JP)

(74) Representative: Henkel, Feiler, Hänzel & Partner
Möhlstrasse 37
D-8000 München 80(DE)

## (54) Aminoguanidine derivatives.

(57) A aminoguanidine derivatives of the general formula:

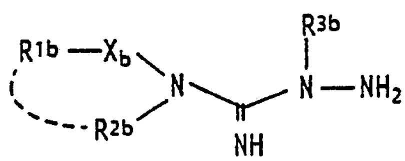

(IB)

(wherein, $R^{1b}$ represents a hydrogen atom, alkyl group of from 1 to 6 carbon atom(s), cycloalkyl group of from 5 to 7 carbon atoms or carbocyclic or heterocyclic ring unsubstituted or substituted by from one to three halogen atom(s), alkyl group(s) of from 1 to 4 carbon atom(s), alkoxy group(s) of from 1 to 4 carbon atom(s) or nitro group, $X_b$ represents a bond, alkylene group of from 1 to 4 carbon atom(s) or alkenylene group of from 2 to 4 carbon atoms,
$R^{2b}$ represents a hydrogen atom, alkyl group of from 1 to 6 carbon atom(s) or phenyl group,
$R^{3b}$ represents a hydrogen atom, phenyl group, alkyl group of from 1 to 4 carbon atom(s), phenylalkyl group of from 7 to 10 carbon atoms or pyridyl group

EP 0 339 496 A2

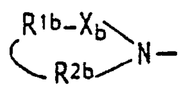

represents a heterocyclic ring, which contains nitrogen atom or one nitrogen and one oxygen atom, of from 3 to 10 carbon atoms unsubstituted or substituted by a hydroxy group.

With the proviso that the compounds wherein $R^{2b}$ represents a hydrogen atom, $-X_b-R^{1b}$ represents a hydrogen atom, methyl group, ethyl group or phenyl group and $R^{3b}$ represents a hydrogen atom or methyl group, are excluded.) or acid addition salt thereof possess inhibitory activity on Maillard reaciton, and therefore is useful for treating and/or prevention of several diabetic complications and diseases induced by aging.

# Aminoguanidine Derivatives

## Summary

This invention is related to aminoguanidine derivatives and medicines containing them as active ingredients.

More particularly, this invention is related to

(1) novel aminoguanidine derivatives of the general fomula:

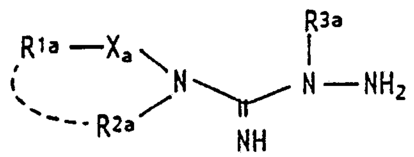

(IA)

(wherein, all of the symbols represent the same meaning as hereinafter defined.),

(2) process for the preparation of them, and

(3) inhibitory agents on Maillard reaction containing the compound of the general formula:

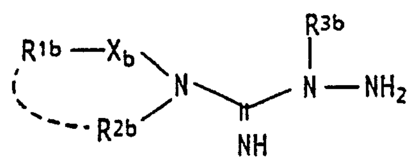

(IB)

(wherein, all of the symbols represent the same meaning as hereinafter defined.) which includes the compounds of the formula (IA), as active ingredient.

## [Background]

In 1912, Maillard reported that, when a mixture of amino acid and reducing sugar is heated, it shows brown color (called browning phenomenon).
[Maillard, L.C., Compt. Rend. Soc. Biol., 72, 599 (1912)], and suggested that this reaction might occur in a body.

In 1968, Rahbar reported that HbAlc, the glycosylated part of haemoglobin (Hb), was increased in diabetics [Rahbar, S., Clin. Chim. Acta., 22, 296 (1968)].

Afterwards, it became clean that HbAlc had the chemical structure in which glucose binds to valine in N-terminal side of $\beta$-chain in the form of Amadori rearrangement [Koenig, R.J., Blobstein, S.H., & Cerami, A., J. Biol. Chem., 252, 2992 (1977)],
and that Maillard reaction proceeds nonenzymatically [Stevens, V.J., Vlassara, H., Abati, A., & Cerami, A., J. Biol. Chem., 252, 2998 (1977)]. These results showed that Maillard reaction occurs in a body.

As an initial step, Maillard reaction consists of forming the Amadori rearrangement products by glycosylation of reducing sugar with amino-group of protein. In the advanced stage of this reaction

① cross-linked compounds [called advanced glycosylation products (abbreviated as "AGE")] are produced,
② solubility of them becomes low,
③ the products can not be easily degraded by the action of proteases,
④ a fluorescent is formed, and then
⑤ the products are colored brown.

The mechanism of AGE production has been proposed by some groups. For example, the theory of Brownlee et al is shown below [Brownlee, M. et al., Science, 232, 1629 (1986)].

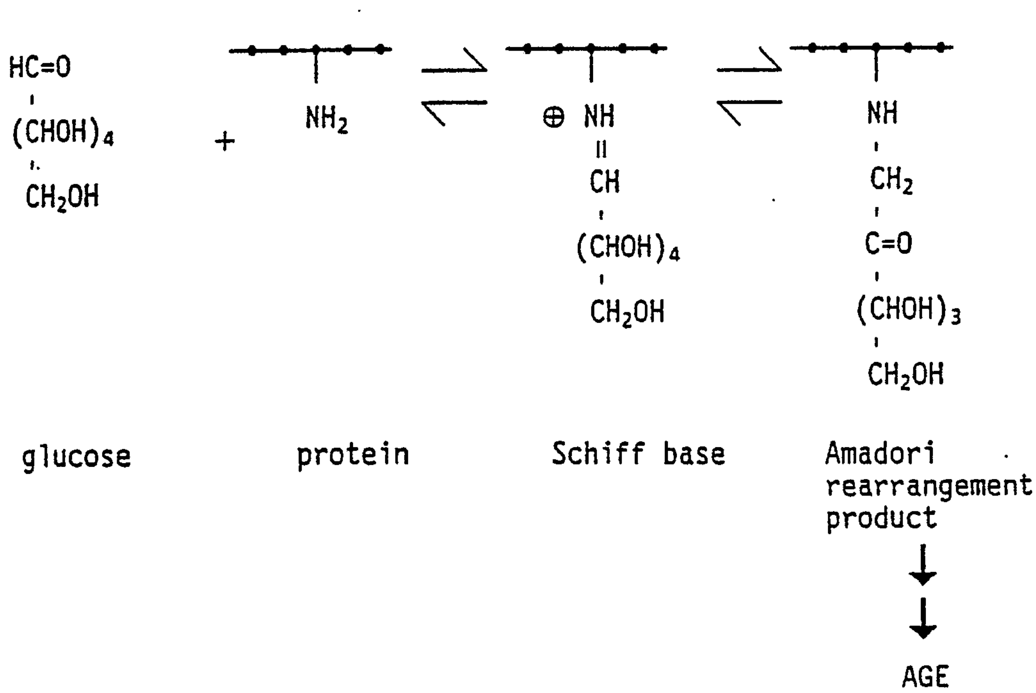


Maillard reaction is observed in healthy person, and particulary this is caused notably in diabetics that a level of blood sugar is high and in protein of which metabolic turnover is slow.

For example, in the case of haemoglobin, the level of glycosylation in diabetic mouse are 2.7 times as high as that of normal mouse [Monnier, V.M. et al., The Maillard Reaction in Foods and Nutrition, ACS Symposium Series, 215, 432, Am. Chem. Soc., Washington, D.C. (1983)], and glycosylation of serum albumin in diabetics was advanced [Guthrow, C.E. et al., Proc. Natl. Acad. Sci. U.S. 76, 4258 (1979)].

Further, it was turned out that, when a glycosylated serum protein was administrated intravenously during 12 weeks, the typical diabetic renal lesion was observed [Monnier, V.M. et al., Clin. Endocrinol. Metab., 11, 431 (1982)].

Crystallin in lens is the special protein which is not metabolized at all after its biosynthesis.
When crystallin was glycosylated, its steric structure was transformed and the SH groups were oxidized to form S-S bond. A sequence of processes induced to polymerize the protein in crystallin.

In the case of diabetic cataract in rats, the ratio of binding of protein and glucose was ten times as high as that of normal rat, and also intramolecular S-S bond formation increased [Monnier, V.M. & Cerami, A. Clin. Endocrinol. Metab. 11, 431 (1982)].

Glycosylation of crystallin caused its polymerization, decrease in its solubility, formation of fluorescent substance, yellow and brown coloring. This phenomenon is very similar to that of lens by aging Chiou, S.H., Chylack, L. T., Jr., Tung, W.H., & Bunu, F., J. Biol. Chem. 256, 5176 (1981)].

Collagen and elastin in connective tissue contain lysine and hydroxylysine abundantly, the speed of their metabolic turnover is slow, and the existance of reactants with glucose at the basement membrance of renal adrenal glands, skin and tendon has been found [Monnier, V.M., Stevens, V.J., & Cerami, A., Maillard Reactions in Food, Prog. Food Nutr. Sci. 5, 315, Pergamon Press, London], further these glycosylation products might be related to sclerosis in a blood vessel wall [Rosenburg, H., Modrak, J.B., Hassing, J.M., Al-Turk, W.A., & Stohs, S.J., Biochem.Biophys.Res. Commun, 91, 498 (1979)].

The cause of diabetic neurosis may be nonenzymatic glycosylation of neuro-myelin protein [Monnier, V.M. et al., Clin.Endocrinol.Metab. 11, 431 (1982)].

In this way, Maillard reaction in a body is related to not only various diabetic complication but also a lot of diseases accompanied with aging.

[Prior arts]

On the above background, recently, researches of Maillard reaction inhibitors has been carried out. For example, Brownlee, M. et al. showed that aminoguanidine inhibits Maillard reaction in vitro, and that aminoguanidine, administered to diabetic rats, inhibits diabetes-induced accumulation of advanced

glycosylation end products in arterial wall connective tissue protein [Brownlee, M. et al., Science, 232, 1629 (1986)].

They have considered that the amino group of a nucleophilic hydrazine compound (i.e. the amino group which is bonded to guanidino group in aminoguanidine) blocks reactive carbonyls on early glycosylation products, and inhibits further cross-link formation of chemically reversible Amadori product.

Further, in the specification of Japanese Patent Kokai No. 62-142114 i.e. European Patent Publication No. 222313, it was suggested that the pharmaceutical composition comprising compound having the group containing reactive nitrogen atoms, which enable to react with reactive carbonyls in chemically reversible Amadori product, inhibits the production of advanced glycosylation end products. Concretely, the compositions comprising aminoguanidine, α-hydrazino histidine and lysine are disclosed.

And further, as the compounds which have an equal or similar structure with those of this invention, it is suggested that the compounds of the formula as follows.

① In the specification of United States Patent No. 210,291, it is disclosed that the compounds of the general formula:

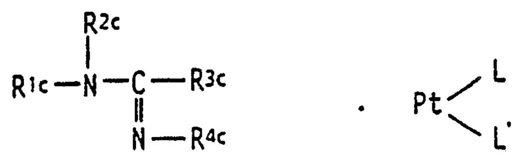

(C)

(wherein, $R^{1c}$ represents an amino group or benzyl group,
$R^{2c}$ represents a hydrogen atom or alkyl group of from 1 to 3 carbon atom(s),
$R^{3c}$ represents an amino, hydrazino, dialkylamino (of from 1 to 3 carbon atom(s), phenylamino, benzylamino, alkylamino (of from 1 to 3 carbon atom(s)), diphenylamino, 2-thienylamino, 2-furanylmethylamino, or 2-,3- or 4-pyridinylmethylamino gorup,
$R^{4c}$ represents a hydrogen atom and L and L′ represents dibasic organic acids.) (the difinitions of the simbols described above are selection.) are useful as antineoplastic agents.

② In the specification of Canadian Patent No. 755,123, it is disclosed that the compounds of the general formula:

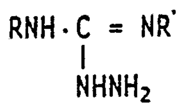

(D)

(wherein, R represents a phenethyl, 3-phenylpropyl or benzyl group substituted by a halogen atom or alkyl group,
R represents a hydrogen atom or lower alkyl group.)
are useful as antihypertensive agents.

③ In the abstract of WPI Accession No. 66-26,286, it is disclosed that the compounds of the general formula:

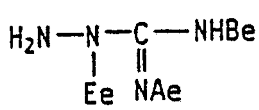

(E)

(wherein, Ae and Ee represent a hydrogen atom, optionally branched alkyl group of from 1 to 6 carbon atom(s) or alicyclic group which are substituted by more than one hydroxy gorup(s),
Be represents a hydrogen atom, alicyclic group which are substituted by more than one hydroxy group(s) or straighted or branched alkyl group of from 1 to 6 carbon atom(s).) (the difinitions of the symbols described above are selection.) are useful as cardiac tonics.

④ In the specification of United States Patent No. 3,377,381, it is disclosed that the compounds of the general formula:

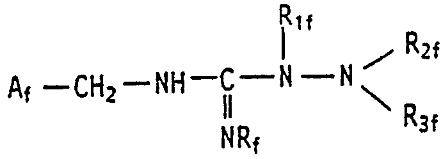

(F)

(wherein, $A_f$ represents a phenyl substituted by halogen in 2-position carbon atom, o-tolyl or naphthyl group, $R_f$, $R_{1f}$, $R_{2f}$ and $R_{3f}$ each represent a hydrogen atom or methyl group.) are useful as antihypertensive agents.

⑤In the specification of United States Patent No. 3972,932, it is disclosed that the compounds of the general formula:

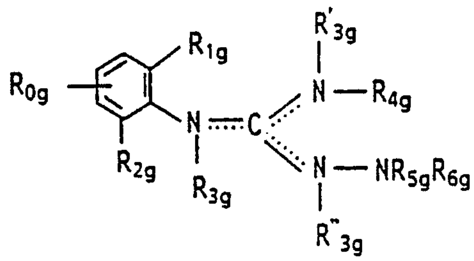

(G)

(wherein, there is a double bond between the C atom of the guanidine group and one of the adjacent N atoms,
$R_{0g}$ is a hydrogen atom, halogen, hydroxy group, alkyl or alkoxy group of from 1 to 6 carbon atoms,
$R_{1g}$ and $R_{2g}$ are halogen atom, hydroxy group, alkyl or alkoxy group of from 1 to 4 carbon atom(s),
$R_{3g}$, $R'_{3g}$ and $R''_{3g}$ are hydrogen atom or alkyl group of from 1 to 4 carbon atom(s) but cannot all be present simultaneously owing to the double bond,
$R_{4g}$ is a hydrogen atom, alkyl or alkoxy group of from 1 to 4 carbon atom(s) , hydroxy group, amino group or mono- or dialkyl of from 1 to 4 carbon atom(s) amino,
$R_{5g}$ and $R_{6g}$ are hydrogen atom, alkyl or acyl group of from 1 to 6 carbon atom(s), or together form an alkylidene, cycloalkylidene or aralkylidene group. With the proviso that $R_{1g}$ and $R_{2g}$ are not both methyl group when $R_{4g}$ is methyl group and $R_{0g}$, $R_{3g}$, $R''_{3g}$, $R_{5g}$ and $R_{6g}$ are all hydrogen atoms.) are usefull as antihypertensive agents.

[Disclosure of the invention]

The present invention is related to

1) novel aminoguanidine derivatives of the general formula:

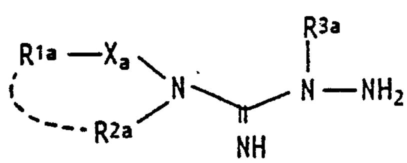

(IA)

(wherein,

(i) $R^{2a}$ and $R^{3a}$ each represent hydrogen atoms, and $-X_a-R^{1a}$ represents a n-butyl, cyclohexyl, benzyl, 4-chlorobenzyl, 4-fluorobenzyl, 4-methoxybenzyl, 4-methylbenzyl, 3-nitrobenzyl, 3,4-dichlorobenzyl, phenethyl, 3-phenyl-2-propenyl or 1-naphthylmethyl group,

(ii) only $R^{2a}$ represents a hydrogen atom and
$-X_a-R^{1a}$ represents a benzyl group and $R^{3a}$ represents a methyl group or
both $-X_a-R^{1a}$ and $R^{3a}$ represent a phenyl group,

(iii) only $R^{3a}$ represents a hydrogen atom and
both $-X_a-R^{1a}$ and $R^{2a}$ represent ethyl groups,
both $-X_a-R^{1a}$ and $R^{2a}$ represent n-butyl groups,
$-X_a-R^{1a}$ represents a benzyl group and $R^{2a}$ represents a phenyl group,
$-X_a-R^{1a}$ represents a phenyl group and $R^{2a}$ represents a methyl group, or
$-X_a-R^{1a}$ represents a benzyl group and $R^{2a}$ represents a methyl group,
(iv) only $R^{3a}$ represents a hydrogen atom and

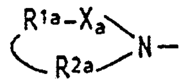

represents a 1-piperidino, 1-(1,2,3,4-tetrahydro) isoquinolyl, 1-pyrrolidinyl, 4-morpholino or 4-hydroxypiperidino group,
(v)

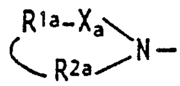

represents a 4-morpholino group and $R^{3a}$ represents a methyl group, or

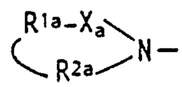

represents a 4-hydroxypiperidino group and $R^{3a}$ represents a propyl group, or
(vi) both $-X_a-R^{1a}$ and $R^{2a}$ represent hydrogen atom and $R^{3a}$ represents benzyl, phenethyl, 3-phenylpropyl, cyclopentylmethyl or 2-pyridyl group.)
and acid addition salts thereof,

2) processes for the preparation of them, and
3) inhibitory agents on Maillard reaction containing the compound of the general formula:

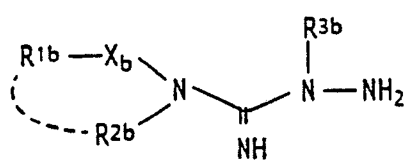

(IB)

(wherein, $R^{1b}$ represents a hydrogen atom, alkyl group of from 1 to 6 carbon atom(s), cycloalkyl group of from 5 to 7 carbon atoms or carbocyclic or heterocyclic ring unsubstituted or substituted by from one to three halogen atom(s), alkyl group(s) of from 1 to 4 carbon atom(s), alkoxy group(s) of from 1 to 4 carbon atom(s) or nitro group, $X_b$ represents a bond, alkylene group of from 1 to 4 carbon atom(s) or alkenylene group of from 2 to 4 carbon atoms, $R^{2b}$ represents a hydrogen atom, alkyl group of from 1 to 6 carbon atom(s) or phenyl group,
$R^{3b}$ represents a hydrogen atom, phenyl group, alkyl group of from 1 to 4 carbon atom(s), phenylalkyl group of from 7 to 10 carbon atoms or pyridyl group

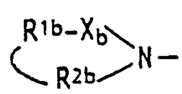

represents a heterocyclic ring, which contains nitrogen atom or one nitrogen and one oxygen atom, of from 3 to 10 carbon atoms unsubstituted or substituted by one hydroxy group. With the proviso that the

compounds wherein $R^{2b}$ represents a hydrogen atom, $-X_b-R^{1b}$ represents a hydrogen atom, methyl group, ethyl group or phenyl group and $R^{3b}$ represents a hydrogen atom or methyl group, are excluded.) which include the compound of the general formula (IA) or acid addition salts thereof as active ingredients.

[Comparison with prior arts]

Aminoguanidine have been considered to inhibit on Maillard reaction because the group containing reactive nitrogen atoms (an amino group bonding to quanidino group) react on reactive carbonyl groups in chemically reversible Amadori Products. However we, inventors of the present invention, have confirmed that the compounds, that substitutes were introduced into whose amino groups, also possess inhibitory activity and already filed an application. This time, inventors of the present invention have found that the compounds, that substitutes were introduced into whose amino groups, also possess inhibitory activity on Maillard reaction and have completed this invention.

Moreover, parts of the compounds of the general formula (IB), of the present invention are equal to the compounds of the general formula (C), (D), (E), (F) and (G). However, the compounds of the general formula (C) was disclosed as antineoplastic agent, the compounds of the general formula (D), (F) and (G) was disclosed as antihypertension agent and the compounds of the general formula (E) was disclosed as cardiac tonics. Accordingly, it can be unexpected that the compounds of the present invention possess an inhibitory action on Maillard reaction over drug efficacies and uses of these prior arts.

Further, the compounds of the general formula (IA) are not concretely disclosed individually.

The present invention includes all isomers unless otherwise specified. For example, alkyl group, alkoxy group, alkylene group and alkenylene group mean straight chain or branched-chain alkyl group, alkoxy group, alkylene group and alkenylene group, and the double-bond in alkenylene group includes E, Z and mixture of E and Z. And, in case of existing branched-chain alkyl group, the present invention includes the isomers caused by existing asymmetrical carbon atoms.

In the general formula (IB), alkyl groups of from 1 to 6 carbon atom(s) shown by $R^{1b}$ and $R^{2b}$ are methyl, ethyl, propyl, butyl, pentyl, hexyl and isomeric group thereof. All groups are preferable. $R^{2b}$ are also preferable to represent a hydrogen atom and phenyl group.

In the general formula (IB), cycloalkyl groups of 5 to 7 carbon atom(s) shown by $R^{1b}$ are cyclopentyl, cyclohexyl and cycloheptyl group. All groups are preferable.

In the general formula (IB), carbocyclic ring shown by $R^{16}$ are mono-, bi or tri-aromatic carbocyclic rings containing not more than 15 carbon atoms which may be partially or fully saturated.

The rings are, for example, benzene, naphthalene, indene, azulene, fluorene, phenanthrene, anthracene, acenaphthylene, biphenylene ring and the rings which may be partially or fully saturated thereof. Preferable rings are benzene and naphtalene ring.

In the general formula (IB), heretocyclic rings in $R^{1a}$ are mono-, bi- or tri- aromatic heterocyclic rings containing not more than 15 carbon and hetero atoms which may be partially or fully saturated. For example, they are furan, thiophene, pyrrole, oxazole, isooxazole, thiazole, isothiazole, imidazole, pyrazole, furazan, pyran, pyridine, pyridazine, pyrimidine, pyrazine, indole, isoindole, benzofuran, benzothiophene, indolizine, chromene, quinoline, isoquinoline, quinolizine, purine, indazole, quinazoline, cinnoline, quinoxaline, phthalazine, pteridine, carbazole, acridine, phenanthridine, xanthene, phenazine, phenothiazine rings, and partially or fully saturated rings thereof.

In the general formula (IB), halogen atoms shown by substituent groups in $R^{1b}$ are fluorine, chlorine, bromine and iodine atom. Alkyl groups of from 1 to 4 carbon atom(s) are methyl, ethyl, propyl, butyl group and isomeric group thereof. Alkoxy groups of from 1 to 4 carbon atom(s) are methoxy, ethoxy, propoxy, butoxy group and isomeric groups thereof. All groups are preferable. Substitutent groups in $R^{1b}$ are also preferable to represent a nitro group. $R^{1b}$ is also preferable to represent unsubstituted carbocyclic and heterocyclic rings.

In the general formula (IB), alkylene groups of from 1 to 4 carbon atom(s) shown by $X_b$ are methylene, ethylene, trimethylene, tetramethylene group and isomeric groups thereof. Alkenylene groups of from 2 to 4 carbon atoms are vinylene, propenylene, butenylene group and isomeric groups thereof. All groups are preferable. $X_b$ is also preferable to represent a bond.

In the general formula (IB), alkyl groups of from 1 to 4 carbon atom(s) shown by $R^{3b}$ are methyl, ethyl, propyl, butyl group and isomeric groups thereof. Phenyl alkyl groups of from 7 to 10 carbon atoms are benzyl, phenethyl, 3-phenylpropyl and 4-phenylbutyl group. All groups are preferable. $R^{3b}$ is also preferable to represent a hydrogen atom, phenyl group and pyridyl group.

In the general formula (IB), heterocyclic rings shown by

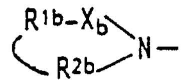 ,

which contains only one nitrogen or one nitrogen and one oxygen atom, of from 3 to 10 carbon atoms are mono-or bi-aromatic heterocyclic rings which may partially or fully saturated thereof. For example, pyrrole, isooxazole, pyridine, isoquinoline, quinoline, pyrrolidine, piperidine, indoline, isoindoline and morpholine ring. These rings are substituted by a hydroxy group or unsubstituted.

And the compounds of the general formula (IA) and (IB), if desired, may be converted into acid addition salts by the known methods. Preferably, acid addition salts are non-toxic salts and water-soluble. The suitable acid addition salts are, for example, hydrochloric acid, hydrobromic acid, hydroiodic acid, sulfuric acid, phosphoric acid, an inorganic acid such as nitric acid, or an organic acid such as acetic acid, lactic acid, tartric acid, benzoic acid, citric acid, methanesulphonic acid, ethanesulphonic acid, benzenesulphonic acid, toluenesulphonic acid, isethionic acid, glucuronic acid and gluconic acid. Acid addition salts may be obtained by the known methods, for example, by reacting stoichiometric quantities of a compound of general formula (IA) and (IB) and the appropriate acid in a suitable solvent.

[Process for the preparation of the compounds of the present invention]

The compounds of the general formula(IA) may be prepared by the method described hereinafter.

That is, it may be carried out by reacting the compounds shown by the general formula:

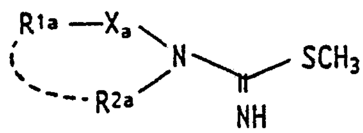 (II)

(wherein, all of the symbols represent the same meanings as hereinbefore defined.) and the compounds of the general formula:

$R^{3a}$-$NHNH_2$ (III)

(wherein, all of the symbols represent the same meanings as hereinbefore defined.) in water or alkanol (e.g. methanol, ethanol), at a room temperature or reacting the compounds of the general formula:

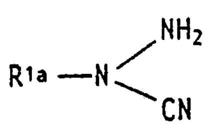 (IV)

(wherein, the symbol represents the same meaning as described hereinbefore.) and ammonia in the presence of ammonium sulfate in water with refluxing.

The compounds of the general formula (IB) may be also prepared by the same method as the compounds of the general formula(IA).

[Starting material]

The starting materials and each reagents of the general formula (II), (III) and (IV) are known or may be prepared by known method.

The reaction products may be purified by conventional methods, for example, distillation at atmospheric or reduced pressure, high performance liquid chromatography, thin layer chromatography or column chromatography using silica gel or magnesium silicate or washing or recrystallization. Purification may be carried out after each reactions or a series of reactions.

[Efficacy]

The compounds of the general formula (IA) and (IB), of the present invention, and acid addition salts thereof possess the inhibitory activity on Maillard reaction. Accordingly, the compounds of the present invention are useful for treatment and/or prevention of several diabetic complication, i.e. coronary heart disease, peripheral circulatory insufficiency or failure, cerebrovascular hindrance, neurogenous diabetes, nephropathy, arteriosclerosis, arthrosclerosis, cataracta and retinopathy, and the diseases induced by aging, i.e. atherosclerosis, senile cataract and cancer.

An inhibitory activity on Maillard reaction of the compounds of the present invention was confirmed by the screening system mentioned below.

(1) Method of experiment

In order to evaluate the inhibitory effect on Maillard reaction in vitro, of the compounds in the present invention, the measurement was carried out under conditions described below.

Bovine serum albumin (100 mg/ml), glucose (200 mM) and test compound (6 mM, 30 mM in example 2 and 2(a)) were dissolved in 0.5 M phosphatic buffer solution (pH 7.38), and the mixture was incubated for a week at 37°C.

After incubation, the incubated medium was diluted 100 times with the same phosphatic buffer solution, and the fluorescence spectrum was measured at the excitation maximum of 360nm and the emission maximum of 450 nm.

Inhibition percentage of the test compounds was calculated by the following equation:

$$\text{Inhibition (\%)} = \frac{\Delta I - \{\Delta I_4 - (\Delta I_1 + \Delta I_2 + \Delta I_3)\}}{\Delta I}$$

$\Delta I_1$ : fluorescence of test compounds

$\Delta I_2$ : fluorescence of (test compounds + glucose),

$\Delta I_3$ : fluorescence of (test compounds + BSA),

$\Delta I_4$ : fluorescence of (test compounds + BSA + glucose),

$\Delta I$ : fluorescence of (BSA + glucose)

(2) Results

The results are shown in the following table I.

Table

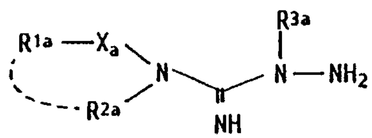 or 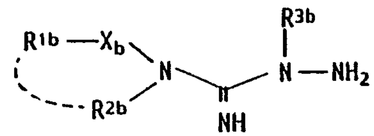

| Example No. | 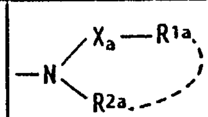 or 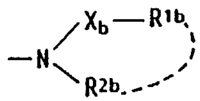 | $R^{3a}$ or $R^{3b}$ | Salts | Name | Inhibitory effect (%) |
|---|---|---|---|---|---|
| 1 | 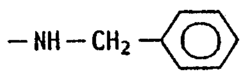 | H | hydroiodide | 1-amino-3-benzylguanidine hydroiodide | 63 |
| 1 (a) | 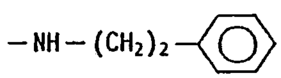 | H | hydroiodide | 1-amino-3-phenethylguanidine hydroiodide | 72 |
| 1 (e) | 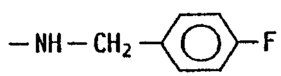 | H | hydroiodide | 1-amino-3-(4-fluorobenzyl) guanidine hydroiodide | 43 |
| 1 (f) | 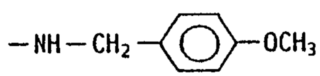 | H | hydroiodide | 1-amino-3-(4-methoxybenzyl) guanidine hydroiodide | 54 |

Table 1 (continued)

| Example No. | 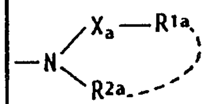 or 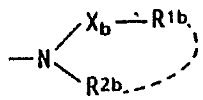 | R3a or R3b | Salts | Name | Inhibitory effect (%) |
|---|---|---|---|---|---|
| 1 (h) | 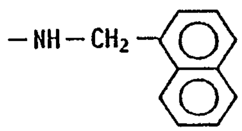 | H | hydroiodide | 1-amino-3-(1-naphtylmethyl) guanidine hydroiodide | 51 |
| 1 (i) | 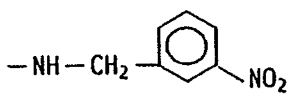 | H | hydroiodide | 1-amino-3-(3-nitrobenzyl) guanidine hydroiodide | 57 |
| 1 (o) | 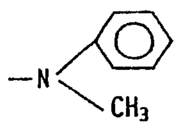 | H | hydroiodide | 1-amino-3-methyl-3-phenyl guanidine hydroiodide | 54 |
| 1 (g) | 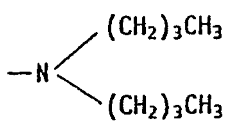 | H | hydroiodide | 1-amino-3,3-n-butylguanidine hydroiodide | 34 |
| 1 (r) | 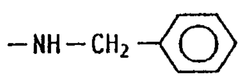 | $CH_3$ | hydroiodide | 1-amino-1-methyl-3-benzylguanidine hydroiodide | 36 |

Table 1 (continued)

| Example No. | 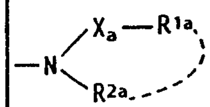 or 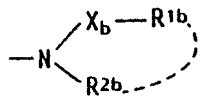 | $R^{3a}$ or $R^{3b}$ | Salts | Name | Inhibitory effect (%) |
|---|---|---|---|---|---|
| 1 (u) | 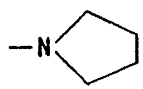 | H | hydrochloride | 1-pyrrolidinecarbohydrazide imide hydrochloride | 43 |
| 1 (v) | 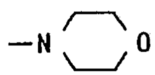 | H | hydroiodide | 4-morpholinecarbohydrazide imide hydrochloride | 36 |
| 2 | $-NH_2$ | 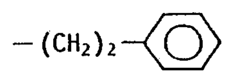 | — | 1-amino-1-phenethylguanidine | 71 |
| 2 (a) | $-NH_2$ | 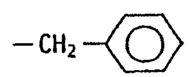 | — | 1-amino-1-benzylguanidine | 50 |
| 2 (b) | $-NH_2$ | 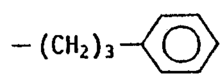 | — | 1-amino-1-(3-phenylpropyl) guanidine | 50 |
| 2 (c) | $-NH_2$ | 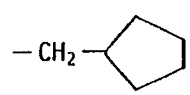 | — | 1-amino-1-cyclopenthylmethyl guanidine | 56 |

The results in the Table I show that the compounds of the present invention and acid addition salts possess an inhibitory effect on Maillard reaction.

[Toxicity]

It was confirmed that the toxicity of the compounds of the present invention were very low.

According it was confirmed that the compounds of the present invention were useful for prevention and/or treatment for deseases attributed to Maillard reaction, in animals including human beings, especially human beings.

[Administration]

For the purpose above described, the compounds of the present invention of the general formula (IA), (IB) and acid addition salts thereof may normally by administered systemically or partially, usually by oral or parenteral administration.

The doses to be administered are determined depending upon age, body weight, symptom, the desired therapeutic effect, the route of administration, and the duration of the treatment etc. In or acid addition the human adult, the doses per person per dose are generally between 1 mg and 1000 mg and 100 mg, by parenteral administration (preferably, intravenous administration) up to several times per day.

As mentioned above, the doses to be used depend upon various conditions. Therefore, there are cases in which doses lower than or greater than the ranges specified above may be used.

Solid compositions for oral administration in the present invention, include compressed tablets, pills, dispersible powders, and granules. In such compositions, one or more of the active compound(s) is or are, admixed with at least one inert diluent (hydroxypropylcellulose, microcrystalline cellulose, starch, polyvinylpyrrolidone, magnesium metasilicate aluminate etc.). The compositions may also comprise, as is normal practice, additional substances other than inert diluents: e.g. lubricating agents (magnesium stearate etc.), disintegrating agents (cellulose calcium gluconate etc.), stabilizing agent (lactose etc.), and assisting agent for dissolving (glutamic acid, aspertic acid etc.).

The tablets or pills may, if desired, be coated with film of gastric or enteric material (sugar, gelatin, hydroxypropylcellulose or hydroxypropylmethyl cellulose phthalate etc.), or be coated with more than two films. And further, it may be include capsules of absorbable materials such as gelatin.

Liquid compositions for oral administration include pharmaceutically-acceptable solutions, emulsions, suspensions, syrups and elixirs.

In such compositions, one or more of the active compound(s) is or are comprise in inert diluent(s) commonly used in the art (purified water, ethanol etc.).

Besides inert diluents, such compositions may also comprise adjuvants (wetting agents, suspending agents etc.), sweetening agents, flavouring agents, perfuming agents and preserving agents.

Other compositions for oral administration include spray compositions which may be prepared by known methods and which comprise one or more of the active compound(s).

Spray compositions may comprise additional substances other than inert diluents: e.g. stabilizing agents (sodium sulfite etc.), isotonic buffer (sodium chloride, sodium citrate, citric acid etc.).

For preparation of such spray compositions, for example, the method described in the United States Patnet No. 2868691 or 3095355 may be used.

Injections for parenteral administration include sterile aqueous or non-aqueous solutions, suspensions and emulsions. In such compositions, one more of active compound(s) is or are admixed at least one of inert aqueous diluent(s) (distilled water for injection, physiological salt solution etc.) or inert non-aqueous diluent(s) (propylene glycol, polyethylene glycol, olive oil, ethanol, POLYSOLBATE 80 (registered trade mark) etc.).

Injections may comprise additional other than inert diluents: e.g. preserving agents, wetting agents, emulsifying agents, dispersing agents, stabilizing agents (lactose etc.), assisting agents such as assisting agents for dissolving (glutamic acid, aspertic acid etc.).

They may be sterillized, for example, by filtration through a bacteria-retaining filter, by incorporation of sterilizing agents in the compositions or by irradiation. They also be manufactured in the form of sterile solid compositions, for example, by freeze-drying, and which can be dissolved in sterile water or some other sterile diluents for injection immediately before used.

Other compositions for parenteral administration include liquids for external use, and endermic linimetns (ointment etc.), suppositories and pessaries which comprise one or more of the active compound(s) and may be prepared by known methods.

The following examples illustrate the compounds of the present invention and the process for the preparation of them, but not limit the present invention.

The solvents in the parentheses show the developing or eluting solvents and the rations of the solvents used are by volume in chromatographic separations. "IR" were measured by KBr tablet method.

## Example 1

1-amino-3-benzylguanidine hydroiodide

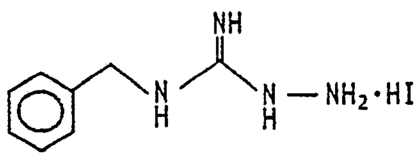

N-benzyl-S-methylthiourea hydroiodide (1.98 g) was dissolved in a mixture of water (6 ml) and a little methanol. An aqueous solution (4 ml) of hydrazine hydride (334 mg) was added to the solution. The mixture was stirred for 1.5 hours at a room temperature. Then the solvent was removed. The residue was dried under reduced pressure to give pale yellow crystals (1.82 g). This crystals was recrystalized from ethanol - water to give pale grey solid (1.18 g). Moreover, this solid was recrystalized from methanol to give the title compound having the following physical data (1.08 mg).
IR : ν 3275, 3125, 1685, 1635 $cm^{-1}$.

## Example 1 (a) - 1 (z)

The compounds of the present invention shown in the following table 2 were obtained with using the corresponding and appropriate compounds of the general formula:

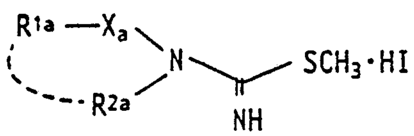

instead of N-benzyl-S-methylisothiourea hydroiodide used in Example 1, by the same procedure as example 1.

With the proviso that the corresponding and appropriate hydrozine derivatives was used in example 1(r) and 1(x) ~ 1(z). And the compounds in example 1(j), 1(k) and 1(s) were converted into hydrochlorides, the compound in 1(l) was converted into sulfate and the compound in 1(y) was converted into acetate.

Table 2

| Example No. | Structual formula | Name | TLC | IR |
|---|---|---|---|---|
| 1 (a) | 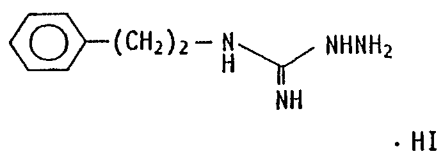 | 1-amino-3-phenethylguanidine hydroiodide | Rf 0.4 (chloroform :methanol = 3:1) | ν 3350 ~ 3150, 1660 ~ 1630, 745, 690 $cm^{-1}$ |
| 1 (b) | 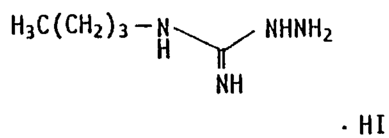 | 1-amino-3-n-butylguanidine hydroiodide | Rf 0.5 (ethyl acetate :acetic acid :water = 3:1:1) | ν 3250 ~ 3150, 2950, 1650 ~ 1640 $cm^{-1}$ |
| 1 (c) | 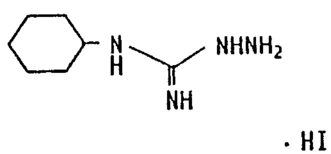 | 1-amino-3-cyclohexylguanidine hydroiodide | Rf 0.51 (ethyl acetate :acetic acid :water = 3:1:1) | ν 3225 ~ 3175, 2925, 1660 ~ 1640 $cm^{-1}$ |
| 1 (d) | 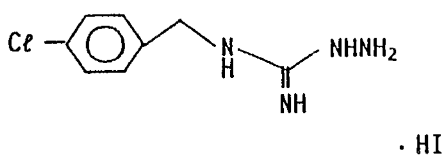 | 1-amino-3-(4-chlorobenzyl) guanidine hydroiodide | Rf 0.22 (chloroform :methanol = 3:1) | ν 3275, 3125, 1680, 1630 $cm^{-1}$ |

Table 2 (continued)

| Example No. | Structual formula | Name | TLC | IR |
|---|---|---|---|---|
| 1 (e) | 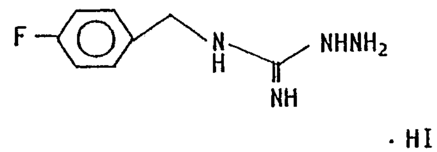 | 1-amino-3-(4-fluorobenzyl) guanidine hydroiodide | Rf 0.25 (chloroform :methanol = 3:1) | ν 3400, 3250, 1680, 1650 $cm^{-1}$ |
| 1 (f) | 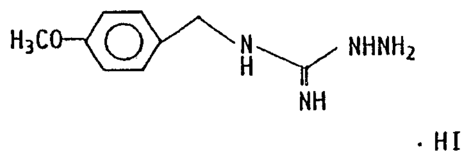 | 1-amino-3-(4-methoxybenzyl) guanidine hydroiodide | Rf 0.6 (chloroform :methanol = 3:1) | ν 3325, 3225, 3150, 1630, 1240 $cm^{-1}$ |
| 1 (g) | 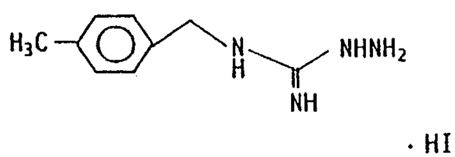 | 1-amino-3-(4-methylbenzyl) guanidine hydroiodide | Rf 0.5 (chloroform :methanol = 3:1) | ν 3400, 3200, 1660, 1630, 530 $cm^{-1}$ |
| 1 (h) | 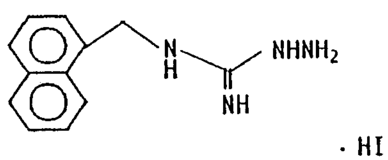 | 1-amino-3-(1-naphthylmethyl) guanidine hydroiodide | Rf 0.59 (ethyl acetate :aceticacid :water = 3:1:1) | ν 3325, 3250~3150, 1640, 790, 760 $cm^{-1}$ |

Table 2 (continued)

| Example No. | Structual formula | Name | TLC | IR |
|---|---|---|---|---|
| 1 (i) | 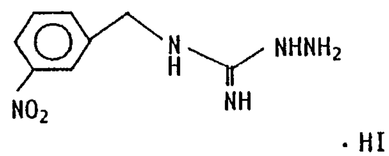 | 1-amino-3-(3-nitrobenzyl) guanidine hydroiodide | Rf 0.24 (chloroform :methanol = 3:1) | ν 3250, 1670, 1640, 1530, 1350, 970, 730 $cm^{-1}$ |
| 1 (j) | 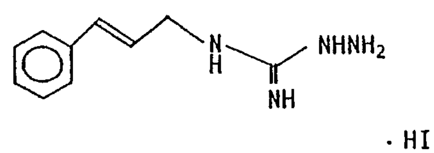 | 1-amino-3-(3-phenyl-2-propenyl) guanidine hydroiodide | Rf 0.25 (chloroform :methanol = 3:1) | ν 3600 ~ 2750, 1620 $cm^{-1}$ |
| 1 (k) | 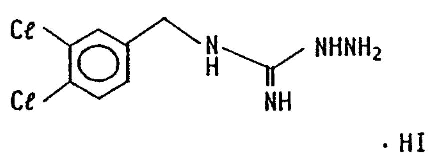 | 1-amino-3-(3,4-dichlorobenzyl) hydroiodide | Rf 0.33 (chloroform :methanol = 3:1) | ν 3400 ~ 2300, 1660, 1640, 1505, 1195, 1020, 930 $cm^{-1}$ |
| 1 (l) | 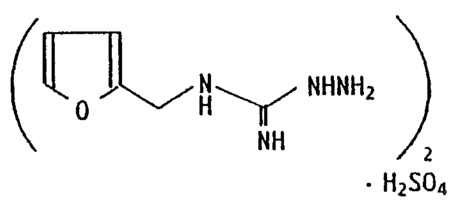 | 1-amino-3-furfurylguanidine sulfate | Rf 0.73 (ethyl acetate :acetic acid :water = 5:1:1) | ν 3600 ~ 2800, 1600 ~ 1610, 1255 ~ 1170, 1000 $cm^{-1}$ |

Table 2 (continued)

| Example No. | Structual formula | Name | TLC | IR |
|---|---|---|---|---|
| 1 (m) | 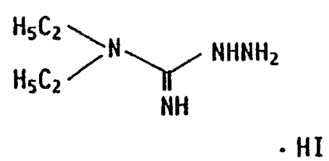 | 1-amino-3,3-diethylguanidine hydroiodide | Rf 0.27 (chloroform :methanol = 3:1) | ν 3450, 3250 ~ 3175, 1620, 1580 $cm^{-1}$ |
| 1 (n) | 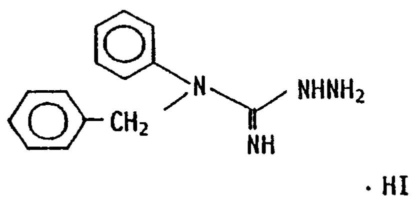 | 1-amino-3-benzyl-3-phenylguanidine hydroiodide | Rf 0.80 (ethyl acetate :acetic acid :water = 3:1:1) | ν 3400, 3250, 3150, 1640, 1620, 1560 $cm^{-1}$ |
| 1 (o) | 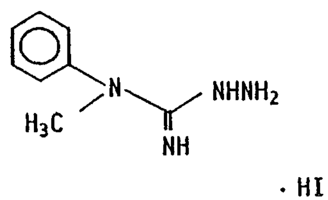 | 1-amino-3-phenyl-3-methylguanidine hydroiodide | Rf 0.57 (ethyl acetate :acetic acid :water = 3:1:1) | ν 3350, 3275, 3175, 1630, 1570, 940, 680 $cm^{-1}$ |
| 1 (p) | 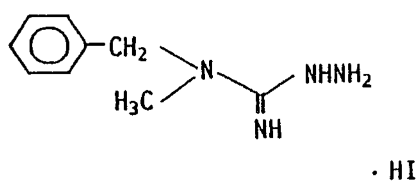 | 1-amino-3-benzyl-3-methylguanidine hydroiodide | Rf 0.59 (ethyl acetate :acetic acid :water = 3:1:1) | ν 3400, 3275, 3220, 1630, 1010, 690, 550 $cm^{-1}$ |

Table 2(continued)

| Example No. | Structual formula | Name | TLC | IR |
|---|---|---|---|---|
| 1 (q) | 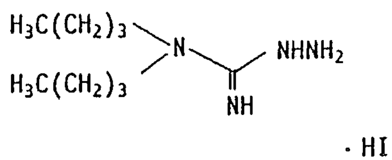 | 1-amino-3,3-di-n-butylguanidine hydroiodide | Rf 0.6 (ethyl acetate :acetic acid :water = 3:1:1) | ν 3400, 3275, 3200, 2975, 1630, 1010, 570 ~ 550 $cm^{-1}$ |
| 1 (r) | 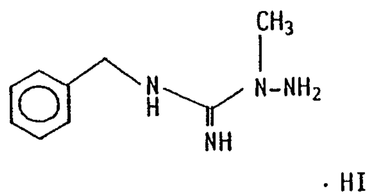 | 1-amino-1-methyl-3-benzylguanidine hydroiodide | Rf 0.2 (chloroform :methanol = 5:1) | ν 3300 ~ 3150, 1660 ~ 1640, 1450, 1020, 750 $cm^{-1}$ |
| 1 (s) | 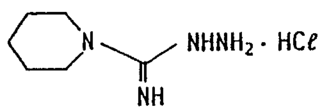 | 1-pyperidinecarbohydrazide imide hydrochloride | Rf 0.2 (chloroform: tetrahydrofuran :acetic acid = 3:1:1) | ν 3600 ~ 2600, 2915, 1640 ~ 1560, 1430, 1230 $cm^{-1}$ |
| 1 (t) | 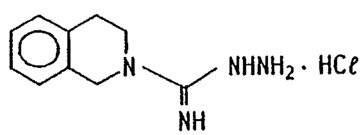 | 1-(1,2,3,4-tetrahydro)isoquinoline carbohydrazide imide hydrochloride | Rf 0.50 (chloroform: tetrahydrofuran :acetic acid = 3:1:1) | ν 3600 ~ 2100, 1660, 1625, 1590, 1440, 1215, 760 $cm^{-1}$ |

Table 2 (continued)

| Example No. | Structual formula | Name | TLC | IR |
|---|---|---|---|---|
| 1 (u) | 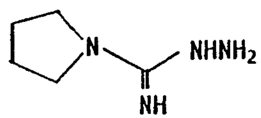 · HCℓ | 1-pyrrolidinecarbohydrazide imide hydrochloride | Rf 0.25 (chloroform: tetrahydrofuran :acetic acid = 3:1:1) | ν 3600 ~ 2000, 1670 ~ 1630, 1570, 1440, 1215, 1090 $cm^{-1}$ |
| 1 (v) | 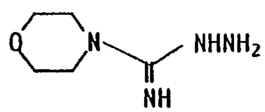 · HCℓ | 4-morpholinecarbohydrazide imide hydrochloride | Rf 0.22 (chloroform: tetrahydrofuran :acetic acid = 3:1:1) | ν 3600 ~ 2400, 1630, 1585, 1430, 1280 1100 $cm^{-1}$ |
| 1 (w) | 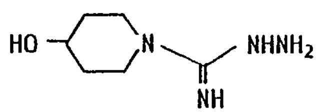 · HCℓ | 1-(4-hydroxy)pyperidinecarbohydrazide imide hydrochloride | Rf 0.33 (chloroform: tetrahydrofuran :acetic acid = 3:1:1) | ν 3650 ~ 2500, 1630, 1590, 1430, 1060 $cm^{-1}$ |
| 1 (x) | 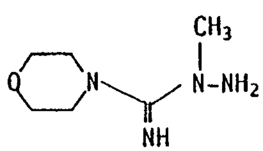 · HCℓ | N'-methyl-4-morpholinocarbohydrazide imide hydrochloride | Rf 0.23 (ethyl acetate :acetic acid :water = 3:1:1) | ν 3650 ~ 2600, 1630, 1610, 1420, 1280, 1105 $cm^{-1}$ |

Table 2 (continued)

| Example No. | Structual formula | Name | TLC | IR |
|---|---|---|---|---|
| 1 (y) | 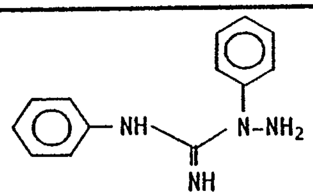 · HI | 1-amino-1-phenyl-3-phenylguanidine hydroiodide | Rf 0.58 (ethyl acetate :acetic acid :water = 10:1:1) | ν 3600 ~ 2400, 1620, 1580, 1485, 1240, 750 $cm^{-1}$ |
| 1 (z) | 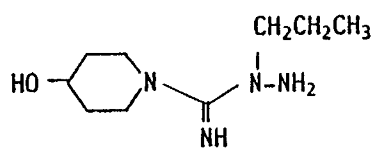 · $CH_3COOH$ | N'-n-poropyl-1-(4-hydroxy) piperidinecarbohydrazide imide acetate | Rf 0.41 (ethyl acetate :aceticacid :water = 3:1:1) | ν 3650 ~ 2600, 2930, 1710, 1620, 1430, 1235, 1055 $cm^{-1}$ |

Example 2

1-Amino-1-phenethylguanidine

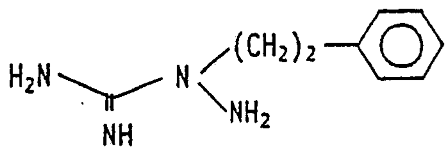

Ammonium sulfate (2.7 g) was dissolved in an aqueous solution (50 ml) of ammonia (10 N) Isopropanol (25 ml) and next N-cyano-N-phenethylhydrazine (3.3 g) were added to the solution. The mixture was refluxing for 2.5 hours. The reaction mixture was cooled to a room temperature. The mixture was separated to two layers. The upper water layer was concentrated. The residue was dried under reduced pressure. The obtained residue was extracted twice with hot ethanol. The extract was concentrated to give the residue like oil. A little ethanol was added to the residue. The produced crystals were added by filtration. The crystals were recrystalized from methanol to give the title compound (586 mg) having the following physical data.
TLC: Rf 0.52 (ethyl acetate : acetic acid : water = 3:1:1);
IR: ν 3300, 1630, 1590, 1570, 1100, 910, 610 $cm^{-1}$.

Example 2 (a) ~ 2 (d)

The compounds of the present invention shown in the following Table 3 were obtained using the corresponding and appropriate hydrazine derivatives instead of N-cyano-N-phenethylhydrazine used in example 2, by the same procedure as example 2. With the proviso that the compound in example 2 (d) was converted into sulfate.

Table 3

| Example No. | Structual formula | Name | TLC | IR |
|---|---|---|---|---|
| 2 (a) | 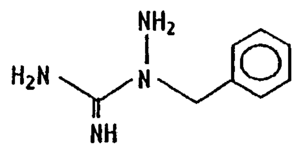 | 1-amino-1-benzylguanidine | Rf 0.50 (ethylacetate :acetic acid :water = 3:1:1) | ν 3350, 1650, 1630, 1595, 1560, 1110, 725, 610 $cm^{-1}$ |
| 2 (b) | 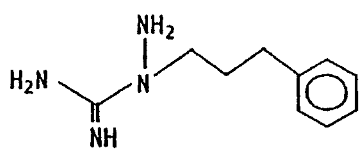 | 1-amino-1-(3-phenylpropyl) guanidine | Rf 0.64 (ethyl acetate :acetic acid :water = 3:1:1) | ν 3600 ~ 2500, 1750 ~ 1400, 1330, 1150 $cm^{-1}$ |
| 2 (c) | 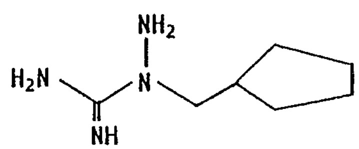 | 1-amino-1-cyclopentylmethyl guanidine | Rf 0.62 (ethyl acetate :acetic acid :water = 3:1:1) | ν 3600 ~ 2500, 2940, 1740 ~ 1370 $cm^{-1}$ |
| 2 (d) | 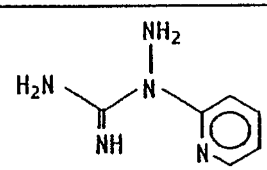 $\cdot H_2SO_4$ | 1-amino-1-(2-pyridyl)guanidine sulfate | Rf 0.56 (chloroform: tetrahydrofuran :acetic acid = 3:1:1) | ν 3400 ~ 2400, 1650, 1625, 1570, 1495, 1410, 1030 $cm^{-1}$ |

[Proparative example]

the following components were admixed in conventional method and punched out to obtain 100 tablets each containing 50 mg of active ingredient.

| | |
|---|---|
| . 1-amino-3-benzylguanidine hydroiodide ..... | 5.0 g |
| . Cellulose Calcium glycolate ..... (distintegrating agent) | 0.2 g |
| . Magnesium stearate ..... (lubricating agent) | 0.1 g |
| . Microcrystaline cellulose ..... | 4.7 g |

## Claims

1) A novel aminoguanidine derivative of the general formula:

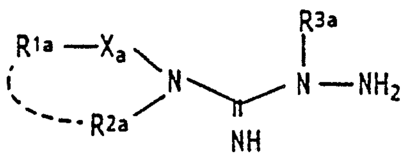

(IA)

(wherein,

(i) $R^{2a}$ and $R^{3a}$ each represent hydrogen atoms, and $-X_a-R^{1a}$ represents a n-butyl, cyclohexyl, benzyl, 4-chlorobenzyl, 4-fluorobenzyl, 4-methoxybenzyl, 4-methylbenzyl, 3-nitrobenzyl, 3,4-dichlorobenzyl, phenethyl, 3-phenyl-2-propenyl or 1-naphthylmethyl group,

(ii) only $R^{2a}$ represents a hydrogen atom and
$-X_a-R^{1a}$ represents a benzyl group and $R^{3a}$ represents a methyl group or
both $-X_a-R^{1a}$ and $R^{3a}$ represent a phenyl group,

(iii) only $R^{3a}$ represents a hydrogen atom and
both $-X_a-R^{1a}$ and $R^{2a}$ represent ethyl groups,
both $-X_a-R^{1a}$ and $R^{2a}$ represent n-butyl groups,
$-X_a-R^{1a}$ represents a benzyl group and $R^{2a}$ represents a phenyl group,
$-X_a-R^{1a}$ represents a phenyl group and $R^{2a}$ represents a methyl group, or
$-X_a-R^{1a}$ represents a benzyl group and $R^{2a}$ represents a methyl group,

(iv) only $R^{3a}$ represents a hydrogen atom and

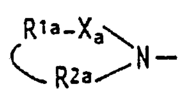

represents a 1-piperidino, 1-(1,2,3,4-tetrahydro) isoquinolyl, 1-pyrrolidinyl, 4-morpholino or 4-hydroxypiperidino group,

(v)

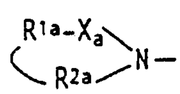

represents a 4-morpholino group and $R^{3a}$ represents a methyl group, or

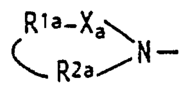

represents a 4-hydroxypiperidino group and $R^{3a}$ represents a propyl group, or

(vi) both $-X_a-R^{1a}$ and $R^{2a}$ represent hydrogen atom and $R^{3a}$ represents benzyl, phenethyl, 3-phenylpropyl, cyclopentylmethyl or 2-pyridyl group.)

2) A compound according to calim 1, which is
1-amino-3-benzylguanidine,
1-amino-3-phenethylguanidine,
1-amino-3-n-butylguanidine,
1-amino-3-cyclohexylguanidine,
1-amino-3-(4-chlorobenzyl)guanidine,
1-amino-3-(4-fluorobenzyl)guanidine,
1-amino-3-(4-methoxybenzyl)guanidine,
1-amino-3-(4-methylbenzyl)guanidine,
1-amino-3-(1-naphtylmethyl)guanidine,
1-amino-3-(3-nitrobenzyl)guanidine,
1-amino-3-(3-phenyl-2-propenyl)guanidine or
1-amino-3-(3,4-dichlorobenzyl)guanidine.

3) A compound according to claim 1, which is
1-amino-1-methyl-3-benzylguanidine or
1-amino-1-phenyl-3-phenylguanidine.

4) A compound according to claim 1, which is
1-amino-3,3-diethylguanidine,
1-amino-3-benzyl-3-phenylguanidine,
1-amino-3-methyl-3-phenylguanidine,
1-amino-3-benzyl-3-methylguanidine or
1-amino-3,3-di-n-butylguanidine.

5) A compound according to claim 1, which is
1-piperidinecarbohydrazide imide,
1-(1,2,3,4-tetrahydro)isoquinoline)carbohydrazide imide,
1-pyrrolidinecarbohydrazide imide,
4-morpholinecarbohydrazide imide or
1-(4-hydroxy)piperidinecarbohydrazide imide.

6) A compound according to claim 1, which is
N′-methyl-4-morpholinecarbohydrazide imide or
N′-n-propyl-1-(4-hydroxy)piperidinecarbohydrazide imide 7) A compound according to claim 1, which is
1-amino-1-phenethylguanidine,
1-amino-1-benzylguanidine,
1-amino-1-(3-phenylpropyl)guanidine,
1-amino-1-cyclopentylmethylguanidine or
1-amino-1-(2-pyridyl)guanidine.

8) A process for the preparation of the aminoguanidine derivatives of the general formula:

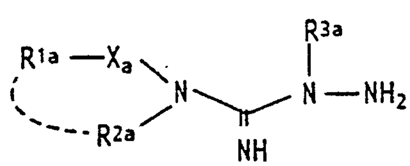 (IA)

(wherein, all of the symbols represent the same meanings as described hereinbefore.) which is characterized by

i) reacting the compound of the general formula:

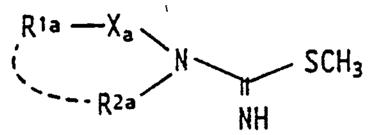

(II)

(wherein, the symbols represent the same meanings as described hereinbefore.) and the compounds of the general formula:

$R^{3a}$-$NHNH_2$ (III)

(wherein the symbols represent the same meanings as described hereinbefore.) or

ii) reacting the compounds of the general formula:

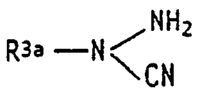

(IV)

(wherein, the symbols represent the same meanings as described hereinbefore.).

9) A pharmaceutical composition, which comprises an effective amount of the aminoguanidine derivatives of the general formula:

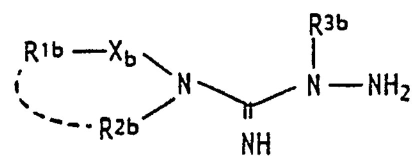

(IB)

(wherein, $R^{1b}$ represents a hydrogen atom, alkyl group of from 1 to 6 carbon atom(s), cycloalkyl group of from 5 to 7 carbon atoms or carbocyclic or heterocyclic ring unsubstituted or substituted by from one to three halogen atom(s), alkyl group(s) of from 1 to 4 carbon atom(s), alkoxy group(s) of from 1 to 4 carbon atom(s) or nitro group, $X_b$ represents a bond, alkylene group of from 1 to 4 carbon atom(s) or alkenylene group of from 2 to 4 carbon atoms,

$R^{2b}$ represents a hydrogen atom, alkyl group of from 1 to 6 carbon atom(s) or phenyl group,

$R^{3b}$ represents a hydrogen atom, phenyl group, alkyl group of from 1 to 4 carbon atom(s), phenylalkyl group of from 7 to 10 carbon atoms or pyridyl group

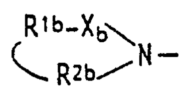

represents a heterocyclic ring, which contains nitrogen atom or one nitrogen and one oxygen atom, of from 3 to 10 carbon atoms unsubstituted or substituted by a hydroxy group.

With the proviso that the compounds wherein $R^{2b}$ represents a hydrogen atom, -$X_b$-$R^{1b}$ represents a hydrogen atom, methyl group, ethyl group or phenyl group and $R^{3b}$ represents a hydrogen atom or methyl group, are excluded.) which include the compound of the general formula (IA) or acid addition salts thereof and pharmaceutically acceptable carrier and/or coating.

10) A pharmaceutical composition according to claim 9, which comprises an effective amount of 1-amino-3-furfurylguanidine.

11) The method for treatment or prevention of several diabetic complications and diseases induced by aging which comprises the administration of an effective amount of the carbazoyl derivative of the general formula (IB).

Claims for the following Contracting States: ES, GR

1. A process for the preparation of the aminoguanidine derivatives of the general formula:

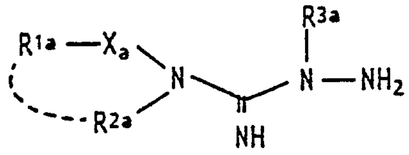

(IA)

(wherein, all of the symbols represent the same meanings as described hereinbefore.) which is characterized by

i) reacting the compound of the general formula:

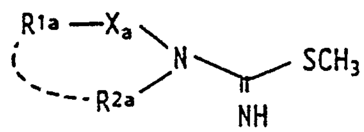

(II)

(wherein, the symbols represent the same meanings as described hereinbefore.) and the compounds of the general formula:

$R^{3a}$-$NHNH_2$ (III)

(wherein the symbols represent the same meanings as described hereinbefore.) or

ii) reacting the compounds of the general formula:

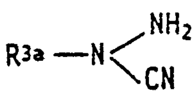

(IV)

(wherein, the symbols represent the same meanings as described hereinbefore.).